# EUROPEAN PATENT SPECIFICATION

(11) **EP 0 005 944 B1**
(45) Date of publication and mention of the grant of the patent: **16.03.1983**
(21) Application number: 79300868.1
(22) Date of filing: 18.05.1979
(51) Int. Cl.: C07C 127/19, C07C 149/437, A01N 47/28

(54) **Substituted benzoylimidates, their preparation and compositions comprising them for insecticidal use**
Substituierte Benzimidosäure-Derivate, ihre Herstellung und sie enthaltende Zusammensetzungen zu insektizider Verwendung
Benzimidates substitués, leur préparation et compositions à usage insecticide les contenant

(30) Priority: 02.06.1978 GB 2629978
(43) Date of publication of application: 12.12.1979
(73) Proprietor: IMPERIAL CHEMICAL INDUSTRIES PLC, London SW1P 3JF (GB)
(72) Inventor: Huff, Roger Kenneth, Nr Newark Nottinghamshire (GB); Evans, David Daniel, Binfield Berkshire (GB); Anderson, Nicholas Hugh, Wokingham Berkshire (GB)
(74) Representative: Bishop, Nigel Douglas

## Description

This invention relates to chemical compounds useful in the control of pests, particularly insect pests, to compositions containing the compounds, and to methods of preparing the compounds.

1-Phenyl-3-halobenzoylureas having insecticidal properties are known from J. Ag. Food Chem 24 (₁) pages 134-136 (1976) and 1-halophenyl-3-benzimidoylureas useful as veterinary anthelmintic agents are described in US Patent 3547937.

According to the present invention there are provided compounds of the formula (I): 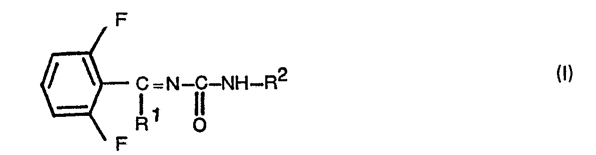 wherein R¹ is an alkenyloxy group; an alkoxy group optionally substituted by one or more phenyl radicals or atoms of fluorine, chlorine, bromine or iodine; a phenoxy group optionally substituted by one or more lower alkyl or lower alkoxy groups or atoms of fluorine, chlorine, bromine or iodine; an alkylthio group optionally substituted by one or more phenyl radicals; a phenylthio radical optionally substituted by one or more atoms of fluorine, chlorine, bromine or iodine, or lower alkyl, lower alkoxy, or lower haloalkyl radicals; or an amino radical optionally substituted by one or two alkyl radicals, each of which may optionally be substituted by one or more alkoxy radicals or phenyl radicals; and R² is a phenyl radical optionally substituted by one or more atoms of fluorine, chlorine, bromine or iodine or by one or more lower haloalkyl radicals, or by one or more phenoxy radicals optionally substituted by one or more atoms of fluorine, chlorine, bromine, or iodine, or by one or more lower haloalkyl groups.

By the terms lower alkyl, lower alkoxy, and lower haloalkyl groups, we mean groups in which the alkyl moiety contains from 1 to 6 carbon atoms.

Examples of compounds according to the invention include those of the foregoing formula (I) in which the group R² is a 3,4-dichlorophenyl or 4-chloro-3-trifluoromethyl phenyl radical, and R¹ is an alkoxy group of 1 to 12 carbon atoms.

Particular examples of compounds provided by the invention are listed in Table I below:

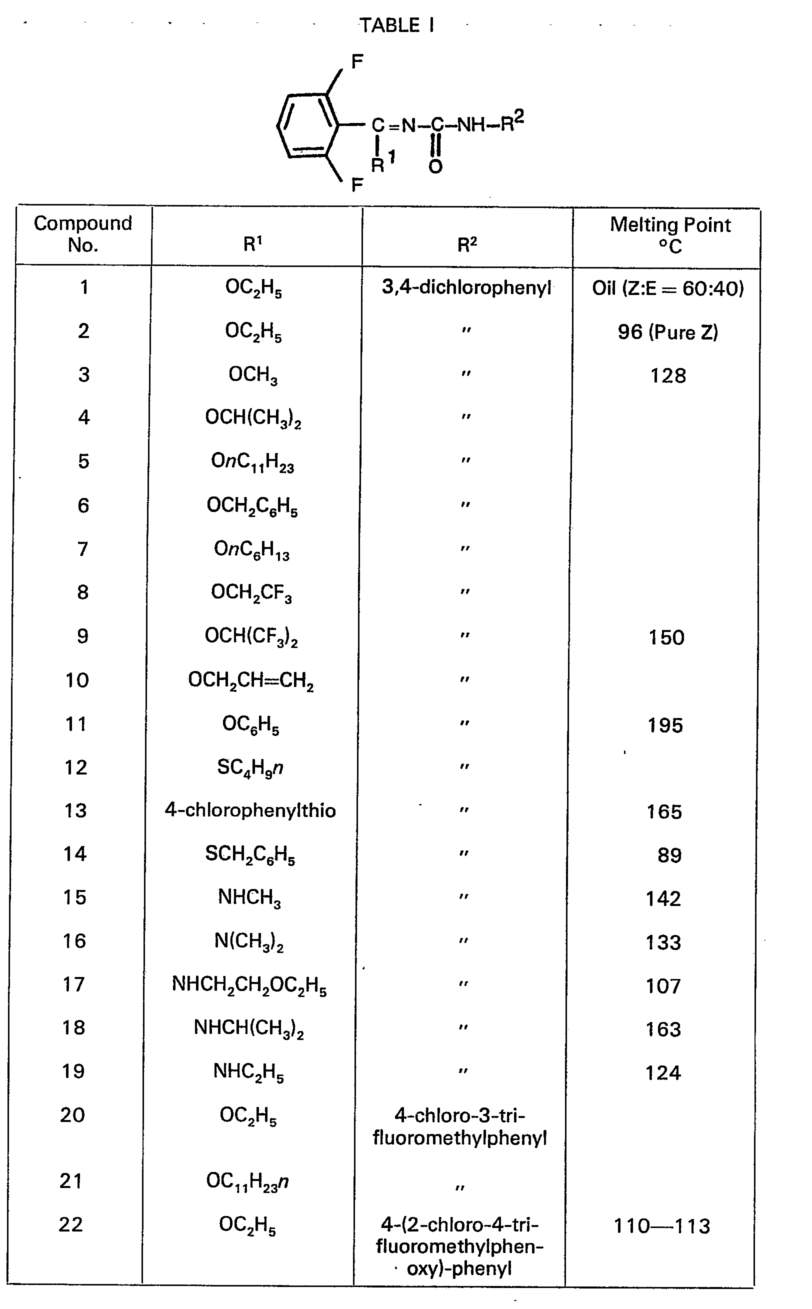

The compounds of the invention are capable of existing in two different geometrically isomeric forms, depending upon the spatial disposition of the substituent groups about the C=N bond in the molecule. Following conventional chemical nomenclature, the two forms of a particular compound are designated as the E and the Z isomers of the compound. Referring to compound no. 1 of Table I, the E and Z forms are shown below by way of example. 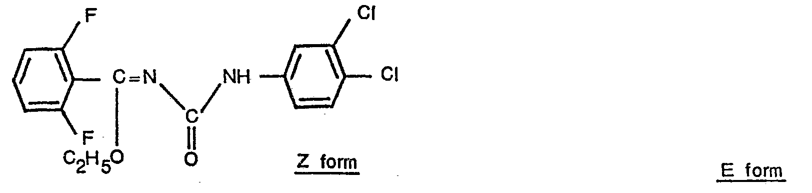 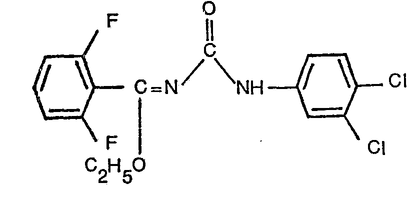 In Table I, compounds 1 and 2 have the same molecular formula. Compound 1 is a mixture of E and Z forms in the ratio shown and compound 2 is the pure Z isomer.

Depending upon the process chosen for preparing a particular compound, or on the way in which the conditions for a particular process are varied, either of the E and Z forms may be obtained, or a mixture of the two. The isomers, having different physical properties, may be separated by physical processes known in the chemical art. Both isomers of a particular compound have biological activity, but the biological effects of the isomers may not be completely identical in every case. The compounds in Table I are mixtures of E and Z forms unless otherwise specified.

The compounds of formula I may be used to combat and control infestations of insect pests particularly lepidopterous and coleopterous insects. The insect pests which may be combated and controlled by the use of the invention compounds include those pests associated with agriculture (which term includes the growing of crops for food and fibre products, horticulture and animal husbandry), forestry, the storage of products of vegetable origin, such as fruit, grain timber, and also those pests associated with the transmission of diseases of man and animals. Thus the compounds may be used to control mosquito larvae and the larvae of the common housefly. The compounds may also be effective in controlling infestations of Coleoptera and Diptera by causing the laying of infertile eggs.

In order to apply the compounds to the locus of the pests they are usually formulated into compositions which include in addition to the insecticidally active ingredient or ingredients of formula I suitable inert diluent or carrier materials, and/or surface active agents. The compositions may also comprise another pesticidal material, for example another insecticide or acaricide, or a fungicide, or may also comprise an insecticide synergist, such as for example dodecyl imidazole, safroxan, or piperonyl butoxide.

The compositions may be in the form of dusting powders wherein the active ingredient is mixed with a solid diluent or carrier, for example kaolin, bentonite, kieselguhr, or talc, or they may be in the form of granules, wherein the active ingredient is absorbed in a porous granular material for example pumice.

Alternatively the compositions may be in the form of liquid preparations to be used as dips or sprays, which are generally aqueous dispersions or emulsions of the active ingredient in the presence of one or more known wetting agents, dispersing agents or emulsifying agents (surface active agents).

Wetting agents, dispersing agents and emulsifying agents may be of the cationic, anionic or non-ionic type. Suitable agents of the cationic type include, for example, quaternary ammonium compounds, for example, cetyltrimethyl ammonium bromide. Suitable agents of the anionic type include, for example, soaps, salts of aliphatic monoesters or sulphuric acid, for example sodium lauryl sulphate, salts of sulphonated aromatic compounds, for example sodium dodecylbenzenesulphonate, sodium, calcium or ammonium lignosulphonate, butyl naphthalene sulphonate, and a mixture of the sodium salts of diisopropyl- and triisopropylnaphthalene sulphonates. Suitable agents of the non-ionic type include for example, the condensation products of ethylene oxide with fatty alcohols such as oleyl alcohol or cetyl alcohol, or with alkyl phenols such as octyl phenol, nonyl phenol and octyl cresol. Other non-ionic agents are the partial esters derived from long chain fatty acids and hexitol anhydrides, the condensation products of the said partial esters with ethylene oxide, and the lecithins.

The compositions may be prepared by dissolving the active ingredient in a suitable solvent, for example, a ketonic solvent such as diacetone alcohol, or an aromatic solvent such as trimethylbenzene and adding the mixture so obtained to water which may contain one or more known wetting, dispersing or emulsifying agents. Other suitable organic solvents are dimethyl formamide, ethylene dichloride, isopropyl alcohol, propylene glycol and other glycols, diacetone alcohol, toluene, kerosene, white oil, methylnaphthalene, xylenes and trichloroethylene, N-methyl-2-pyrrolidone and tetrahydro furfuryl alcohol (THFA).

The compositions to be used as sprays may also be in the form of aerosols wherein the formulation is held in a container under pressure in the presence of a propellant such as fluorotrichloromethane or dichlorodifluoromethane. The compositions which are to be used in the form of aqueous dispersions or emulsions are generally supplied in the form of a concentrate containing a high proportion of the active ingredient or ingredients, the said concentrate to be diluted with water before use. These concentrates are often required to withstand storage for prolonged periods and after such storage, to be capable of dilution with water to form aqueous preparations which remain homogeneous for a sufficient time to enable them to be applied by conventional spray equipment. The concentrates may contain 10-85% by weight of the active ingredients. When diluted to form aqueous preparations, such preparations may contain varying amounts of the active ingredient depending upon the purpose for which they are to be used.

For agricultural or horticultural purposes, an aqueous preparation containing between 0.0001% and 0.1% by weight of the active ingredient is particularly useful.

In use the compositions are applied to the pests, to the locus of the pests, to the habitat of the pests, or to growing plants liable to infestation by the pests, by any of the known means of applying pesticidal compositions, for example, by dusting or spraying. The rate of application will depend upon factors such as the insect species to be controlled but in general a rate of from 5 to 1000 g per hectare will be appropriate.

The compositions of the invention are very toxic to a variety of insect pests, including, for example, the following:-
Plutelia maculipennis (diamond back moth, larvae)
Phaedon cochleariae (mustard beetle)
Trialeuroides spp (white flies)
Spodoptera littoralis (cotton leaf worm)

Compounds of the invention wherein the group R' is an alkenyloxy, alkoxy, or phenoxy group may be prepared by reacting a 1,3,5-oxadiazine derivative (II) with the appropriate alkenol, alkanol, or phenol.

The reaction is illustrated below for the case where the alkanol is ethanol.

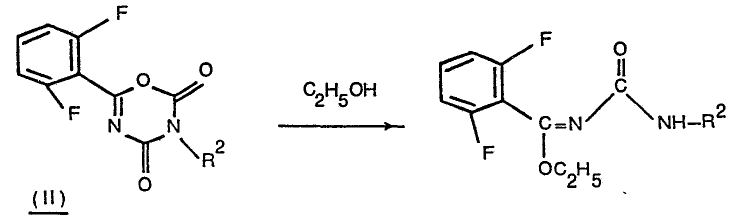

Compounds according to the invention wherein the group R¹ is an alkylthio or phenylthio group may be prepared in the same way by reacting the appropriate 1,3,5-oxadiazine derivative (II) with the appropriate alkanethiol or thiophenol.

Compounds according to the invention in which the group R¹ is an amino group or an amino .group substituted by one or two alkyl groups may be prepared by reacting the appropriate 1,3,5-oxadiazine (ll) with ammonia or with the appropriate mono- or di-alkylamine. The 1,3,5-oxadiazine derivatives (fl) may be prepared as described in German Offenlegungschrift no. 2732115. The reaction with the alkenol, alkanol, phenol, alkanethiol, thiophenol, ammonia, or amine may if desired be carried out in a solvent inert to the reactants. It may be convenient to carry out the reaction by treating the oxadiazine with an excess of the other reactant as the solvent. The reaction may if desired be accelerated by heating.

In an alternative method of preparation, compounds in which R¹ is an alkoxy or alkenyloxy group may be prepared by the reaction scheme outlined below: 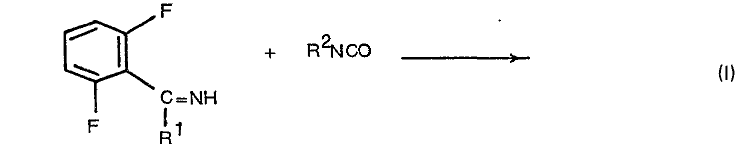 In this scheme, the imidate ester (III), wherein R¹ is an alkenyloxy, or alkoxy group, is reacted with the appropriate isocyanate R²NCO to give the compounds of the invention. The imidate esters and the isocyanates R²NCO may be prepared by standard methods well known in the chemical art. Compounds wherein the group R¹ is an alkylthio group may also be prepared by the last foregoing reaction scheme.

The invention is illustrated by the following Examples in which all the parts are by weight and all temperatures in degrees Centigrade unless otherwise specified.

### Example 1

This Example illustrates the preparation of compound no. 20 of Table I.

### (a) Preparation of ethyl 2,6-difluorobenzimidate

2,6-Difluorobenzamide (1.56 g) in methylene chloride was added to triethyloxonium fluoborate (1.9 g) in methylene chloride and the mixture stirred for 24 hours. The solution was then washed three times with cold sodium carbonate solution and dried over sodium sulphate. The methylene chloride solution was evaporated to yield an oil. This was mixed with chloroform and the solution filtered from insoluble material. Evaporation of the chloroform gave the ethyl 2,6-difluorobenzimidate as a mobile oil.

### (b) Preparation of compound no. 20

The product from paragraph (a) above (0.33 g) in dry ether was added slowly to a stirred solution of 4-chloro-3-trifluoromethylphenyl isocyanate (0.28 g) in dry ether. The mixture was stirred for several hours and then left at room temperature for 3 days. The solvent was then removed and the residue recrystallised from hexane to give compound no. 20 with a melting point of 109°C.

### Example 2

### This Example illustrates the preparation of compound no. 2 of Table I (i.e. the pure Z isomer of compound no. 1).

3 - (3,4 - Dichlorophenyl) - 6 - (2,6 - difluorophenyl) - 3,4 - dihydro - 2 - H - 1,3,5 - oxadiazin - 2,4 - dione (0.6 g) prepared as described in Example 3 of West German Offenlegungschrift no. 2732115, was heated under reflux with ethanol (15 ml) for 2 hours, when all the solid had dissolved. Water was then added until the solution became slightly turbid and the solution left to stand. The crystals which separated were collected and identified as compound no. 2, having a melting point of 96°C. The nuclear magnetic resonance spectrum of the product was in agreement with the structure assigned.

### Example 3

This Example illustrates the insecticidal activity of compounds according to the invention.

The compounds of the invention were formulated for test by dissolving a sample of each compound in the minimum amount of a mixture of 1 part by volume of ethyl alcohol and 1 part by volume of acetone, and then diluting the solution so obtained with water to give a solution having a concentration of 1000 parts per million. These solutions were then used in tests on mustard beetle (Phaedon cochleariae) and cotton leaf worm (Spodoptera littoralis). These tests were carried out as follows:

### Mustard Beetle

Pots containing mustard cotyledons are sprayed to run off with the spray solution decribed above. When dry the sprayed foliage is fed to mustard beetle larvae (4th instar stage) kept in Petri dishes containing filter paper (10 larvae per dish). After 48 hours the filter paper is changed and the dead larvae counted. The larvae are fed again with a single unsprayed leaf and further mortality counts made at 6 and 12 days.

### Cotton Leaf Worm Test

The test is similar to that described for the mustard beetle above, with the following differences: Cabbages are used as the test plants and 5 larvae instead of 10 are used. Mortality counts are made at 48 hours at which stage the filter paper is removed and the larvae fed on an untreated artificial diet. A further count is made at 6 days. Two replicates are used in all tests.

The results are given below in Table II.

In the Table, the results given are expressed on a scale of 0 to 3, corresponding to the following ranges of percentage kill:

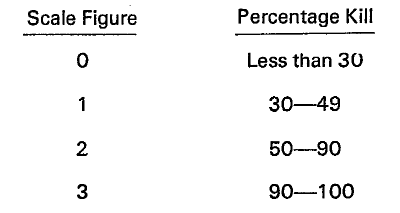

A dash (-) means that no test was carried out.

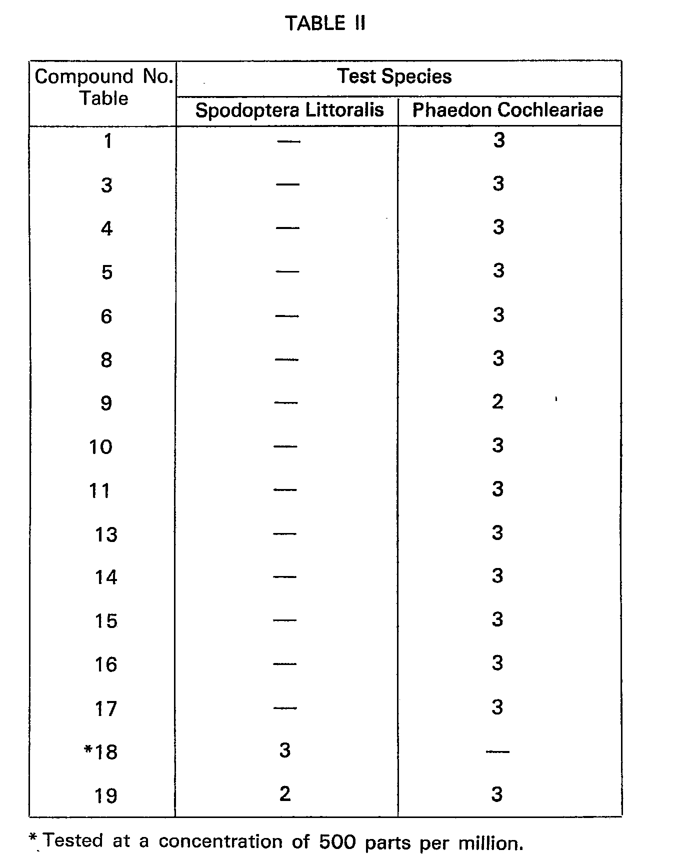

Experiments were carried out in which the compounds of the invention were tested side by side on the foregoing insect species with the known insecticidal compound difluobenzuron (J. Agr. Food Chem. Vol. 24/1 (1976) page 135, compound 19), having the following formula: 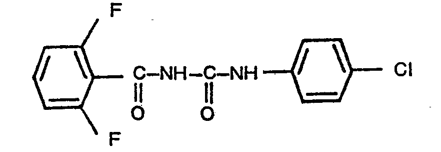 In these tests, a number of the compounds of the invention were substantially more effective than difluobenzuron. Particularly insecticidally active compounds include numbers 1,3,20 and 22 of Table I.

## Claims

1. A compound of the formula (I): 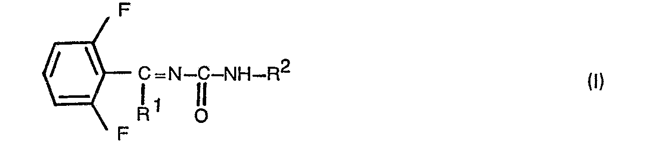 wherein R¹ is an alkenyloxy group; an alkoxy group optionally substituted by one or more phenyl radicals or atoms of fluorine, chlorine, bromine or iodine; a phenoxy group optionally substituted by one or more lower alkyl or lower alkoxy groups or atoms of fluorine, chlorine, bromine or iodine; an alkylthio group optionally substituted by one or more phenyl radicals; a phenylthio radical optionally substituted by one or more atoms of fluorine, chlorine, bromine or iodine, or lower alkyl, lower alkoxy, or lower haloalkyl radicals; or an amino radical optionally substituted by one or two alkyl radicals, each of which may optionally be substituted by one or more alkoxy radicals or phenyl radicals; and R² is a phenyl radical optionally substituted by one or more atoms of fluorine, chlorine, bromine or iodine or by one or more lower haloalkyl radicals, or by one or more phenoxy radicals optionally substituted by one or more atoms of fluorine, chlorine, bromine, or iodine, or by one or more lower haloalkyl groups, wherein said lower alkyl, lower alkoxy or lower haloalkyl groups contain from 1 to 6 carbon atoms.

2. A compound according to claim 1 wherein R¹ is alkoxy of 1 to 12 carbon atoms, and R² is 3,4-dichlorophenyl or 4-chloro-3-trifluoromethylphenyl.

3. A compound according to claim 1 in the form of an isomer of either the E or the Z configuration and substantially free from contamination by the other isomer.

4. The compound according to claim 1 of formula:

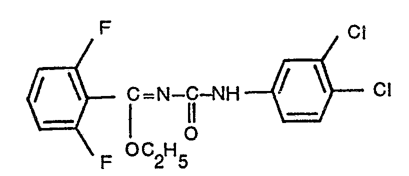

5. The compound according to claim 1 of formula:

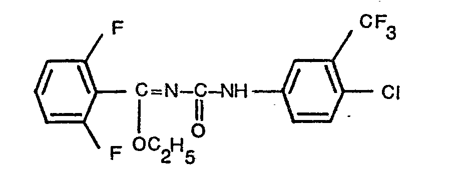

6. The compound according to claim 1 of formula:

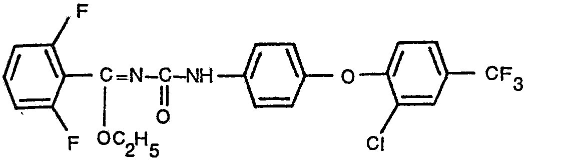

7. An insecticidal composition comprising an insecticidally effective amount of a compound according to claim 1 in association with an inert dilutent or carrier material.

8. A method of combating insect pests in which an insecticidally effective amount of a composition according to claim 7 is applied to the pests, the locus of the pests, the habitat of the pests, or to growing plants liable to infestation by the pests.

9. A process for preparing a compound according to claim 1 which comprises the step of reacting a compound of formula: 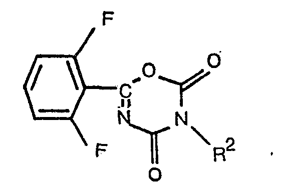 with a compound of formula R^{I}H.

10. A process according to claim 9 carried out in an excess of the compound of formula R^{I}H.

11. A process for preparing a compound according to claim 1 which comprises the step of reacting a compound of formula: 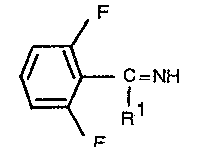 with a compound of formula R²NCO.

## Revendications

1. Un composé de formule (I): 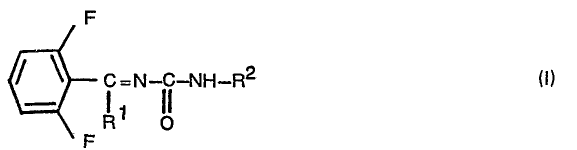 dans laquelle R¹ est un groupe alcényloxy; un groupe alkoxy éventuellement substitué par un ou plusieurs radicaux phényle ou atomes de fluor, chlore, brome ou iode; un groupe phénoxy éventuellement substitué par un ou plusieurs groupes alkyle inférieurs ou alkoxy inférieurs ou atomes de fluor, chlore, brome ou iode; un groupe alkylthio éventuellement substitué par un ou plusieurs radicaux phényle; un radical phénylthio éventuellement substitué par un ou plusieurs atomes de fluor, chlore, brome ou iode ou radicaux alkyle inférieurs, alkoxy inférieurs ou halogénalkyle inférieurs; ou un radical amino éventuellement substitué par un ou deux radicaux alkyle, dont chacun peut éventuellement être substitué par un ou plusieurs radicaux alkoxy ou radicaux phényle; et R² est un radical phényle éventuellement substitué par un ou plusieurs atomes de fluor, chlore, brome ou iode ou par un ou plusieurs radicaux halogénalkyle inférieurs, ou par un ou plusieurs radicaux phénoxy éventuellement substitués par un ou plusieurs atomes de fluor, chlore, brome ou iode, ou par un ou plusieurs groupes halogénalkyle inférieurs, lesdits groupes alkyle inférieurs, alkoxy inférieurs ou halogénalkyle inférieurs contenant 1 à 6 atomes de carbone.

2. Composé suivant la revendication 1, dans lequel R¹ est un groupe alkoxy ayant 1 à 12 atomes de carbone, et R² est un groupe 3,4-dichlorophényle ou 4-chloro-3-trifluorométhylphényle.

3. Composé suivant la revendication 1, sous la forme d'un isomère de configuration E ou de configuration Z et sensiblement dépourvu de contamination par l'autre isomère.

4. Composé suivant la revendication 1, de formule:

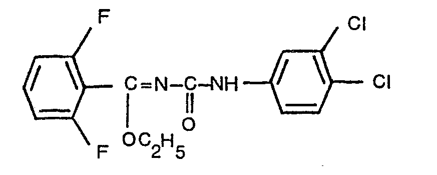

5. Composé suivant la revendication 1, de formule:

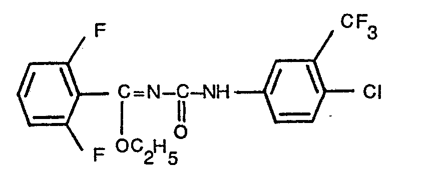

6. Composé suivant la revendication 1, de formule:

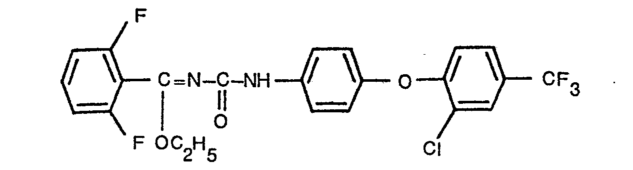

7. Composition insecticide, comprenant une quantité à effet insecticide d'un composé suivant la revendication 1 en association avec un diluant ou support inerte.

8. Procédé de lutte contre des insectes parasites, dans lequel une quantité à effet insecticide d'une composition suivant la revendication 7 est appliquée aux parasites, au lieu infesté de parasites, à l'habitat des parasites ou à des plantes en cours de croissance susceptibles d'une infestation par les parasites.

9. Procédé de préparation d'un composé suivant la revendication 1, qui consiste à faire réagir un composé de formule: 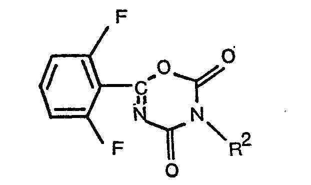 avec un composé de formule R^{I}H.

10. Procédé suivant la revendication 9, mis en oeuvre dans un excès du composé de formule R¹H.

11. Procédé de préparation d'un composé suivant la revendication 1, qui consiste à faire réagir un composé de formule: 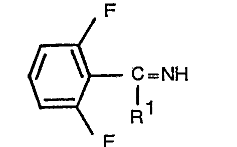 avec un composé de formule R²NCO.

## Patentansprüche

1. Verbindung der Formel (I) 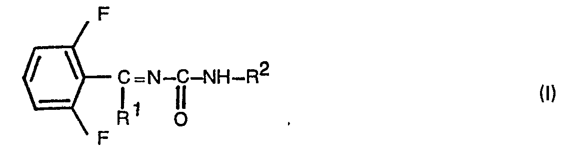 worin R¹ für eine Alkenyloxygruppe; eine Alkoxygruppe, die gegebenenfalls durch ein oder mehrere Phenylradikale oder Fluor-, Chlor-, Brom- oder Jodatome substituiert ist; eine Phenoxygruppe, die gegebenenfalls durch ein oder mehrere Niederalkyl- oder Niederalkoxygruppen oder Fluoro-, Chloro-, Brom- oder Jodatome substituiert ist; eine Alkylthiogruppe, die gegebenenfalls durch ein oder mehrere Phenylradikale substituiert ist; ein Phenylthioradikal, das gegebenenfalls durch ein oder mehrere Fluor-, Chlor-, Brom- oder Jodatome oder Niederalkyl-, Niederalkoxy- oder Niederhalogenoalkylradikale substituiert ist; oder ein Aminoradikal, das gegebenenfalls durch ein oder zwei Alkylradikale substituiert ist, von denen jedes gegebenenfalls durch ein oder mehrere Alkoxyradikale oder Phenylradikale substituiert sein kann, steht; und R² für ein Phenylradikal steht, das gegebenenfalls durch ein oder mehrere Fluor-, Chlor-, Brom- oder Jodatome oder durch ein oder mehrere Niederhalogenoalkylradikale oder durch ein oder mehrere Phenoxyradikale, die ihrerseits durch ein oder mehrere Fluor-, Chlor-, Brom- oder Jodatome oder durch ein oder mehrere Niederhalogenoalkylgruppen substituiert sein können, substituiert ist; wobei die Niederalkyl-, Niederalkoxy- oder Niederhalogenoalkylgruppen 1 bis 6 Kohlenstoffatome enthalten.

2. Verbindung nach Anspruch 1, worin R¹ für Alkoxy mit 1 bis 12 Kohlenstoffatomen steht und R^{2.} für 3,4-Dichlorophenyl oder 4-Chloro-3-trifluoromethylphenyl steht.

3. Verbindung nach Anspruch 1, welche in Form eines Isomers mit entweder der E- oder der Z-Configuration vorliegt und weitgehend frei von Verunreinigung durch das andere Isomer ist.

4. Verbindung nach Anspruch 1 der Formel:

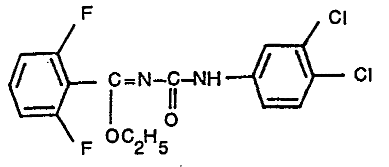

5. Verbindung nach Anspruch 1 der Formel:

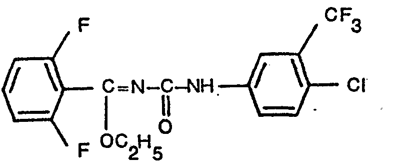

6. Verbindung nach Anspruch 1 der Formel:

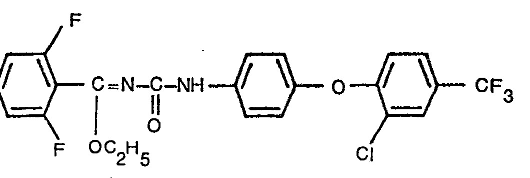

7. Insektizide Zusammensetzung, welche eine insektizid wirksame Menge einer Verbindung nach Anspruch 1 gemeinsam mit einem inerten Verdünnungs- oder Trägermaterial enthält.

8. Verfahren zur Bekämpfung von Insektenschädlingen, bei welchem ein insektizid wirksame Menge einer Zusammensetzung nach Anspruch 7 auf die Schädlinge, auf den Aufenthaltsort der Schädlinge oder auf den Wohnort der Schädlinge oder auf wachsende Pflanzen, denen ein Befall durch die Schädlinge droht, aufgebracht wird.

9. Verfahren zur Herstellung einer Verbindung nach Anspruch 1, bei welchem eine Verbindung der Formel 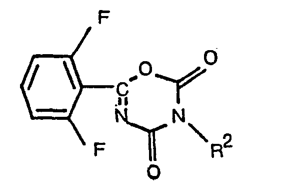 mit einer Verbindung der Formel R¹H umgesetzt wird.

10. Verfahren nach Anspruch 9, welches in einem Überschuß der Verbindung der Formel R¹H ausgeführt wird.

11. Verfahren zur Herstellung einer Verbindung nach Anspruch 1, bei welchem eine Verbindung der Formel 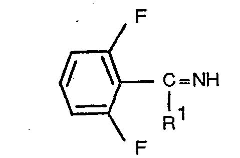 mit einer Verbindung der Formel R²NCO umgesetzt wird.
